(19) Europäisches Patentamt
European Patent Office
Office européen des brevets

(11) Veröffentlichungsnummer : **0 447 758 B1**

(12) **EUROPÄISCHE PATENTSCHRIFT**

(45) Veröffentlichungstag der Patentschrift :
**03.08.94 Patentblatt 94/31**

(51) Int. Cl.$^5$ : **C07C 45/60,** C07C 45/62, C07C 49/203, C07C 49/04

(21) Anmeldenummer : **91100945.4**

(22) Anmeldetag : **25.01.91**

# (54) Verfahren zur Herstellung von ungesättigten und gesättigten Carbonylverbindungen.

(30) Priorität : **06.02.90 DE 4003423**

(43) Veröffentlichungstag der Anmeldung :
**25.09.91 Patentblatt 91/39**

(45) Bekanntmachung des Hinweises auf die Patenterteilung :
**03.08.94 Patentblatt 94/31**

(84) Benannte Vertragsstaaten :
**BE CH DE FR GB LI NL**

(56) Entgegenhaltungen :
**EP-A- 0 141 569**
**EP-A- 0 159 532**
**EP-A- 0 283 359**
**BE-A- 695 674**
**GB-A- 647 971**
**US-A- 3 798 259**
**US-A- 3 812 190**
**US-A- 4 465 863**

(56) Entgegenhaltungen :
**THE CHEMICAL SOCIETY OF JAPAN, Band 60, Mai 1987, S. 1721-1726, The Chemical Society of Japan; J.-I. ISHIYAMA et al: "Chemoselectivity of group-VIII metal catalysts in hydrogenation of nonconjugated enones"**
**CHEMICAL ABSTRACTS, Band 92, Nr. 13, März 1980, Seite 614, Zusammenfassung Nr. 110486v, Columbus, Ohio, US; S. WATANABE et al: "Synthesis of allylketones, alpha, beta-unsaturated ketones, and methylketones from carboxylic acides"**

(73) Patentinhaber : **BASF Aktiengesellschaft**
**Carl-Bosch-Strasse 38**
**D-67063 Ludwigshafen (DE)**

(72) Erfinder : **Merger, Franz, Dr.**
**Max-Slevogt-Strasse 25**
**W-6710 Frankenthal (DE)**
Erfinder : **Fouquet, Gerd, Dr.**
**Kaiserstuhl 34**
**W-6730 Neustadt (DE)**
Erfinder : **Witzel, Tom, Dr.**
**Bruderweg 27**
**W-6700 Ludwigshafen (DE)**
Erfinder : **Becker, Rainer, Dr.**
**Im Haseneck 22**
**W-6702 Bad Duerkheim (DE)**

**EP 0 447 758 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von im Patentanspruch definierten ungesättigten und gesättigten Carbonylverbindungen der allgemeinen Formel I

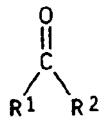

I .

Bisher bekannte ungesättigte und gesättigte Carbonylverbindungen der Formel I dienen als Zwischenprodukte für die Synthese von Duftstoffen und Pharmazeutika.

Aus Comptes R., 120, 1270 (1895) sind Carbonylverbindungen I bekannt, bei denen der Rest $R^1$ einen 3-Methyl-buten-1-ylrest und $R^2$ eine Methylgruppe bedeuten, und ungesättigte Carbonylverbindungen I mit einer Ethylgruppe als Rest $R^2$ und einem 3-Ethyl-buten-1-ylrest als Rest $R^1$ sind in J. Org. Chem., 47, 1267 (1982) beschrieben. Comptes R., 140, 152 (1905) betrifft die Herstellung von Carbonylverbindungen I, in denen $R^1$ einen 3-Methyl-but-1-ylrest und $R^2$ eine Methylgruppe bedeuten.

Gesättigte Carbonylverbindungen I mit einer Ethylgruppe als Rest $R^2$ und einem 3-Ethyl-but-1-ylrest als Rest $R^1$ wurden bisher durch Umsetzung von 4-Methylhexansäurechlorid mit Cadmiumdiethyl (Synthesis, 209 (1979)) oder aus 2,4,6,6-Tetramethyl-dihydro-1,3-oxazin und 1-Iod-2-methylbutan sowie Butyllithium unter Bildung des 2-(3-Methyl-pentyl)-oxazin-Derivates und dessen Reaktion mit Methyliodid und Ethylgrignard (J. Org. Chem., 37, 4259 (1972)) hergestellt.

Die US-A 4 465 863 lehrt Verbindungen I mit einer Butenylgruppe als Rest $R^1$ und einer verzweigten Alkylgruppe als Rest $R^2$, hergestellt in einer vielstufigen Reaktion aus verzweigten Olefinen durch Epoxidierung, Öffnung des Epoxids zum Keton, Reaktion zum entsprechenden 1,3-Diketon, Alkylierung dieses Ketons und Abspaltung einer Keto-Gruppe.

Die EP-A-141 569 lehrt Mischungen von 2,7-Dimethyl-4-oxa-octenen, in denen sich die Doppelbindung in Position 5, 6 oder 7 befindet. Die Herstellung erfolgt durch Umsetzung von Isobutyraldehyd mit 2-Methylpentan-4-on und anschließende Wasserabspaltung.

Aus der EP-A 159 532 ist die Herstellung von 7-Phenyl-1-hepten-5-on und 1-Undecen-5-on durch Reaktion von (ω-Phenylethyl)methylketon bzw. Hexylmethylketon mit Orthoameisensäureester zum Acetal bekannt, gefolgt von einer Umsetzung mit Alkylalkohol bei 150°C unter Druck, wobei eine Umlagerung zu den genannten Produkten eintritt.

Carbonylverbindungen mit einer Butylgruppe als Rest $R^1$ sowie einer Heptyl-, Ethoxy-carbonylpropyl- bzw. Ethoxycarbonylbutylgruppe werden nach der EP-A 283 359 durch Grignard-Reaktion von Butylmagnesiumchlorid mit den entsprechenden Säurechloriden hergestellt.

Die GB-A 647 371 betrifft die Umsetzung von Isobutyraldehyd und Isopren in Gegenwart von Dibenzoylperoxid zu Isopentenylpropylketon.

Die US-A 3 798 259 beschreibt die Reaktion von 2-Alkanoylbernsteinsäuren mit Borsäure bei 120-250°C zu γ-Ketoestern der Formel I, in denen der Rest $R^1$ für Butyl steht.

3,7-Dimethyl-l-octen-5-on kann nach Chemical Abstracts 92 (1980)

13, 110 486v, durch Reaktion einer Vinyl-Grignard-Verbindung mit 6-Methyl-trans-2-hepten-4-on erhalten werden.

Die BE-A-695 674 betrifft die Herstellung von Carbonylverbindungen I, in denen $R^1$ für Butyl und $R^2$ für 2-Methylhexyl bzw. 2-Ethylhexyl steht, durch Dehydrierung des entsprechenden Alkohols an einem Katalysator aus Nickel, Zinkoxid und einem Phosphat.

Diese bisher bekannten Verfahren vermögen jedoch aufgrund des hohen technischen Aufwandes oder ihrer Beschränkung auf nur wenige verschiedene Reste $R^1$ und $R^2$ nicht zu befriedigen.

Der Erfindung lag deshalb die Aufgabe zugrunde, diesen Mängeln abzuhelfen.

Demgemäß wurde ein neues Verfahren zur Herstellung der eingangs definierten ungesättigten und gesättigten Carbonylverbindungen I gefunden, welches dadurch gekennzeichnet ist, daß man ein im Patentanspruch definiertes 2-Formyl-dihydropyranderivat der Formel II

a) zur Gewinnung der ungesättigten Carbonylverbindungen in Gegenwart eines Metalls der VIII. Gruppe des Periodensystems oder einer Verbindung eines dieser Metalle als Katalysatoren, bei 100 bis 400°C umsetzt und

b) zur Gewinnung der gesättigten Carbonylverbindungen die nach Stufe a) erhältlichen ungesättigten Carbonylverbindungen in an sich bekannter Weise hydriert.

Das erfindungsgemäße Verfahren läßt sich wie folgt veranschaulichen:

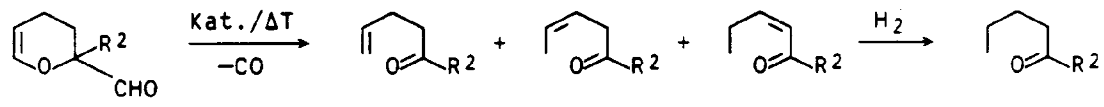


Die erfindungsgemäp einzusetzenden 2-Formyl-dihydropyranderivate II sind bekannt oder nach bekannten Methoden erhältlich.

Die im erfindungsgemäßen Verfahren einzusetzenden Katalysatoren sind Metalle der VIII. Gruppe des Periodensystems oder Verbindungen eines dieser Metalle.

Man kann die Katalysatoren in metallischer Form, in Form von Salzen und Oxiden oder in Form von Komplexverbindungen einsetzen.

Als Katalysatoren in metallischer Form eignen sich besonders Nickel und die Edelmetalle, darunter vor allem Palladium.

Vorzugsweise verwendet man die Metalle in Form von Trägerkatalysatoren auf Trägern wie Aluminiumoxid, Siliciumoxid, Titanoxid, Zinkoxid, Lanthanoxid, Zirkonoxid, Bariumsulfat, Aluminiumsilikat oder Gemischen dieser Materialien sowie vor allem Kohle, wobei Palladium auf Kohle besonders hervorzuheben ist.

Die Trägerkatalysatoren haben vorzugsweise einen Gehalt an aktiven Metallen der VIII. Gruppe von 0,01 bis 50, besonders 0,05 bis 30 und ganz besonders von 1 bis 20 Gew.-%, bezogen auf die Summe aus Träger und katalytisch aktiven Metallen, berechnet als Metall.

Die Katalysatoren können wahlweise als 2- bis 4-mm-Stränge oder als Tabletten mit 3 bis 5 mm Durchmesser, als Splitt mit Teilchengrößen von 0,05 bis 1 mm, vorzugsweise 0,1 bis 0,5 mm als größten Durchmesser oder als Pulver mit Teilchengrößen von 0,05 bis 0,5 mm eingesetzt werden, wobei sich das Pulver auch als Wirbelkontakt eignet.

Verwendet man die definitionsgemäßen Katalysatormetalle in Form ihrer Verbindungen, so eignen sich hierbei besonders Komplexverbindungen des Cobalts, Rutheniums und Rhodiums, beispielsweise Rhodiumacetylacetonat sowie Komplexe mit mindestens zwei Liganden vom Typ der Triorganylphosporverbindungen und Kohlenmonoxid als weitere Liganden.

Unter den Triorganylphosphorverbindungen seien das Tributylphosphin, das Triphenylphosphit und das Triphenylphosphin als einfache und billige Verbindungen dieser Klasse besonders hervorgehoben.

Beispiele für derartige sehr temperaturstabile Katalysatoren sind:

- $CoH(CO)(PPh_3)_3$
- $CoCl(CO)_2(PPh_3)_2$
- $Ru(CO)_3(PPh_3)_2$
- $Ru(CO)(O_2CCF_3)_2(PPh_3)_3$
- $RhH(CO)(PPh_3)_3$
- $RhCl(CO)(PPh_3)_2$

Zur Erhöhung der Stabilität des Katalysators empfiehlt es sich, den Liganden in freier Form mitzuverwenden, und zwar im Molverhältnis Ligand:Molekül von 5:1 bis 100:1 einzusetzen.

Die Menge der Katalysatoren, die man auch in ihren Mischungen einsetzen kann, wird bei diskontinuierlichen Verfahren vorzugsweise so bemessen, daß man 0,001 bis 10 mol-%, vorzugsweise 0,01 bis 1 mol-% des Metalls in metallischer oder gebundener Form zur Verfügung stellt, bezogen auf die Menge der Verbindung II.

Beim kontinuierlichen Verfahren beträgt die Katalysatorbelastung 0,1 bis 200 kg, vorzugsweise 5 bis 100 kg der Verbindung II pro kg des Metalls.

Man führt den Verfahrensschritt a), der zu den ungesättigten Carbonylverbindungen führt, bei 100 bis 400°C, vorzugsweise bei 130 bis 250°C, und ganz besonders bevorzugt bei 150 bis 200°C durch.

Zweckmäßigerweise wird die Umsetzung bei Normaldruck vorgenommen, jedoch kann man auch bei vermindertem oder leicht erhöhtem Druck arbeiten, also etwa im Bereich von 10 mbar bis 20 bar.

Die Reaktionszeiten betragen normalerweise 1 bis 20, meistens 3 bis 15 Stunden.

Es kann zweckmäßig sein, die Umsetzung unter Mitverwendung von Lösungs-oder Verdünnungsmitteln durchzuführen. Hierbei eignen sich Kohlenwasserstoffe wie Dekalin oder Ether wie Diethylenglykoldiethylether.

Man kann die Reaktion diskontinuierlich oder kontinuierlich vornehmen. So kommt eine Festbettreaktion in Sumpf- oder Rieselfahrweise in der Flüssig-oder Gasphase in Betracht. Außerdem eignen sich die Gasphasenreaktion im Wirbelbett oder die Flüssigphasenreaktion mit löslichen Katalysatoren oder suspendierten Trägerkatalysatoren.

Eine wirtschaftlich sehr vorteilhafte Ausführungsform des erfindungsgemäßen Verfahrens besteht in der Durchführung in flüssiger Phase. 2-Formyl-3,4-dihydropyran II und gegebenenfalls Verdünnungsmittel werden über einen festen Katalysator geleitet oder vorzugsweise in Gegenwart eines suspendierten, festen Katalysators oder eines gelösten Homogenkatalysators erhitzt.

Man kann die Aufarbeitung des Gemisches auf die ungesättigten Verfahrensprodukte in an sich bekannter Weise vornehmen, und zwar nach Abtrennung des Katalysators in der Regel durch fraktionierende Destillation des Reaktionsgemisches.

Anschließend werden die ungesättigten Verfahrensprodukte zur Herstellung der gesättigten Produkte in an sich bekannter Weise mit Wasserstoff an einem Katalysator, vorzugsweise bei 40 bis 60°C, umgesetzt. Als Katalysatoren eignen sich Platin und vorzugsweise Raney-Nickel, gegebenenfalls auf einem Träger. Ganz besonders gute Ergebnisse erzielt man mit Palladium auf Kohle.

Im Hinblick auf die gewünschten Verfahrensprodukte I hat das Verfahren für diejenigen Verbindungen I bzw. II besondere Bedeutung, in denen der Rest $R^2$ für

- Wasserstoff oder eine $C_1$-$C_{20}$-Alkylgruppe steht, die bis zu drei der folgenden Substituenten $R^3$ tragen kann:

$C_3$- $C_{12}$-Cycloalkyl; Phenyl; Naphthyl; 5- oder 6-gliedrige aromatische oder nichtaromatische Heterocyclen; $C_2$- $C_6$-Alkenyl; $C_2$- $C_6$-Alkinyl; $C_1$- $C_{12}$-Alkoxycarbonyl; $C_1$- $C_{12}$-Acyloxy; Amino; Mono-$C_1$-$C_4$-alkylamino; Di-$C_1$-$C_4$-alkylamino; $C_1$- $C_{12}$-Acyl; Di-($C_1$-$C_4$-alkyl)-phosphonyl; Hydroxyl; Cyano; $C_1$- $C_4$-Alkoxy; $C_1$-$C_4$-Alkylthio; 5- oder 6-gliedrige Cycloalkylgruppen, deren Ringkohlenstoffatome durch nichtbenachbarte Sauerstoff- oder Schwefelatome ersetzt sein können.

Ganz besondere Bedeutung ist den Verbindungen Ia beizumessen, in denen der Rest $R^{2a}$ folgende Bedeutung hat:

- Wasserstoff oder $C_1$-$C_{20}$-Alkylgruppen, ausgenommen die unsubstituierte Methyl- und Ethylgruppe, vorzugsweise $C_3$-$C_8$-Alkylgruppen, darunter vorzugsweise $C_3$-$C_5$-Alkylgrupen wie iso-Butyl, sec.-Butyl und tert.-Butyl und besonders n-Propyl, iso-Propyl, n-Butyl und n-Pentyl.

Die Alkylgruppen können ihrerseits bis zu drei Substituenten tragen, und zwar vorzugsweise:

- $C_3$-$C_{12}$-Cycloalkyl, Vorzugsweise $C_3$-$C_8$-Cycloalkyl wie Cyclopropyl, Cyclobutyl, Cyclopentyl, Cycloheptyl, Cyclooctyl und besonders Cyclohexyl;
- Naphthyl und besonders Phenyl;
- 5- oder 6-gliedrige aromatische oder nichtaromatische Heterocyclen wie vorzugsweise Pyridyl;
- $C_2$-$C_6$-Alkenyl, vorzugsweise $C_2$-$C_4$-Alkenyl wie Ethenyl, Propenyl und Butenyl;
- $C_1$-$C_{12}$-Alkoxycarbonyl, vorzugsweise $C_1$-$C_8$-Alkoxycarbonyl, darunter vorzugsweise $C_1$-$C_2$-Alkoxycarbonyl wie Ethoxycarbonyl und ganz besonders Methoxycarbonyl.

Daneben eignen sich

- vorzugsweise $C_2$-$C_6$-Alkinyl und $c_1$-$C_{12}$-Alkoxycarbonyl;
- weiterhin Amino, Mono-$C_1$-$C_4$-alkylamino und Di-$c_1$-$C_4$-alkylamino;
- außerdem $C_1$-$C_{12}$-Acyl, Di-($C_1$-$C_4$-alkyl)-phosphonyl, Hydroxyl, Cyano, $C_1$-$C_4$-Alkoxy, $C_1$-$C_4$-Alkylthio und 5- oder 6-gliedrige Cycloalkylgruppen, deren Ringkohlenstoffatome durch nichtbenachbarte Sauerstoff-oder Schwefelatome ersetzt sein können.

Im Hinblick auf die Carbonylverbindungen Ia bevorzugte 2-Formyl-dihydropyranderivate sind 2-Formyl-3,4-dihydropyrane mit zwei gleichen Resten $R^{2a}$ in 2- und 5-Stellung. Diese sind besonders einfach durch Erhitzen von $\alpha$-substituierten Acroleinen nach Diels-Alder zugänglich (Houben-Weyl, Bd. VII, Teil 1, S. 130-131):

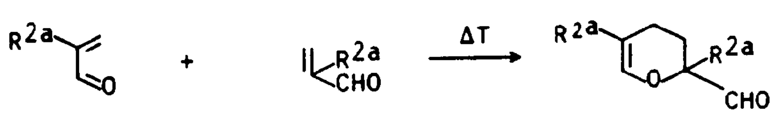


Bevorzugt sind:

- 2,5-Di-n-butyl-2-formyl-3,4-dihydropyran,
- 2,5-Di-n-pentyl-2-formyl-3,4-dihydropyran,
- 2,5-Di(methoxycarbonylmethyl)-2-formyl-3,4-dihydropyran und
- 2,5-Di(1-methoxycarbonylethyl)-2-formyl-3,4-dihydropyran.

Besonders bevorzugt sind:

- 2,5-Di-n-propyl-2-formyl-3,4-dihydropyran,
- 2,5-Di-iso-propyl-2-formyl-3,4-dihydropyran und
- 2,5-Di(3-methoxycarbonylpropyl)-2-formyl-3,4-dihydropyran.

Im Hinblick auf die gewünschten Verfahrensprodukte bevorzugte 2-Formyl-dihydropyranderivate sind 2,5-Dimethyl-2-formyl-3,4-dihydropyran und 2,5-Diethyl-2-formyl-3,4-dihydropyran.

Die nach dem erfindungsgemäßen Verfahren auf einfache und wirtschaftliche Weise erhältlichen ungesättigten und gesättigten Carbonylverbindungen I und Ia eignen sich selber als Duftstoffe oder können als Zwischenprodukte zur Synthese von Duftstoffen und Pharmazeutika verwendet werden. Die ungesättigten Carbonylverbindungen I und Ia können weiterhin als Monomere für die Herstellung von Kunststoffen dienen.

## Beispiele

Herstellung von ungesättigten und gesättigten Carbonylverbindungen der Formel

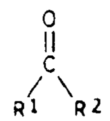

I

a g eines 2-Formyl-3,4-dihydropyrans II und b g Palladium-Katalysator (10 Gew.-% Palladium auf Kohle) wurden für die Dauer von c Stunden auf 155 bis 180°C erhitzt.

Nach Abtrennung des Katalysators wurde das Filtrat fraktionierend destilliert.

Anschließend wurde das ungesättigte Produkt I im Autoklav mit 20 bar Wasserstoff an 0,5 bis 1,3 g Palladium-Kohle (10 Gew.-%) bei 50°C innerhalb von 2 bis 10 Stunden zu dem gesättigten Produkt I hydriert.

Die Einzelheiten dieser Versuche sowie deren Ergebnisse sind der nachstehenden Tabelle zu entnehmen.

Tabelle

Herstellung von ungesättigten und gesättigten Carbonylverbindungen

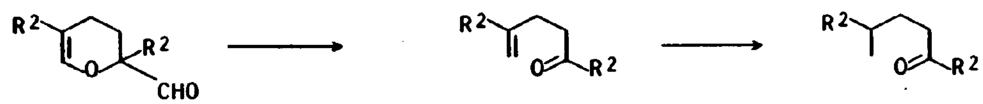

II — unges. I — ges. I

| Bsp. | a (g) | $R^2$ | b (g Pd-Kat.) | c (h) | Ausbeute an unges. I (%) | Ausbeute an ges. I*** (%) | Duftnote der unges. I | Duftnote der ges. I |
|---|---|---|---|---|---|---|---|---|
| 1 | 50,0 | Methyl | 0,50 | 6 | 71,0 | 100 | fruchtig | fruchtig-grün |
| 2 | 500 | Ethyl | 5,0 | 15 | 71,3 | 100 | fruchtig | frisch-blumig |
| 3 | 500 | n-Propyl | 4,5 | 9,5 | 67,9 | 90,5 | grün | fruchtig |
| 4 | 500 | iso-Propyl | 5,0 | 14 | 74,0 | 87,4 | grün | holzig |
| 5* | 50,0 | 3-Methoxycarbonyl-propyl | 1,0 | 5 | ** | 53,0 | schwach holzig | schwach holzig |

* unter Zusatz von 50 g Decalin als Verdünnungsmittel;

** Lösung des Rückstandes des ungesättigten Produktes I in 40 g Tetrahydrofuran;

*** bezogen auf ungesättigtes I.

## Patentansprüche

1. Verfahren zur Herstellung von ungesättigten und gesättigten Carbonylverbindungen der allgemeinen Formel I

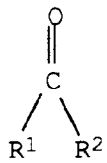

I

in der

$R^1$ einen Buten-1-ylrest oder einen But-1-ylrest bezeichnet, der in 1-, 2-, 3- und/oder 4-Stellung bis zu drei der Reste $R^2$ oder $R^3$ als Substituenten tragen kann und

$R^2$ folgende Substituenten bedeutet:
Wasserstoff oder eine $C_1$-$C_{20}$-Alkylgruppe, die bis zu drei der folgenden Substituenten $R^3$ tragen kann:
$C_3$-$C_{12}$-Cycloalkyl; Phenyl; Naphthyl; 5- oder 6-gliedrige aromatische oder nichtaromatische Heterocyclen;
$C_2$-$C_6$-Alkenyl; $C_2$-$C_6$-Alkinyl; $C_1$-$C_{12}$-Alkoxycarbonyl;
$C_1$-$C_{12}$-Acyloxy; Amino; Mono-$C_1$-$C_4$-alkylamino;
Di-$C_1$-$C_4$-alkylamino; $C_1$-$C_{12}$-Acyl; Di-($C_1$-$C_4$-alkyl)-phosphonyl; Hydroxyl; Cyano; $C_1$-$C_4$-Alkoxy; $C_1$-$C_4$-Alkylthio; 5- oder 6-gliedrige Cycloalkylgruppen, deren Ringkohlenstoffatome durch nichtbenachbarte Sauerstoff- oder Schwefelatome ersetzt sein können,

dadurch gekennzeichnet, daß man ein 2-Formyl-dihydropyranderivat der allgemeinen Formel II

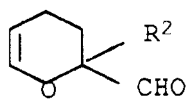

II

bzw. in 3-, 4-, 5- und/oder 6-Stellung durch bis zu drei der Reste $R^2$ oder $R^3$ substituierte Derivate von II

a) zur Gewinnung der ungesättigten Carbonylverbindungen in Gegenwart eines Metalls der VIII. Gruppe des Periodensystems oder einer Verbindung eines dieser Metall als Katalysatoren, bei 100 bis 400°C umsetzt und

b) zur Gewinnung der gesättigten Carbonylverbindungen die nach Stufe a) erhältlichen ungesättigten Carbonylverbindungen in an sich bekannter Weise hydriert.

## Claims

1. A process for the preparation of unsaturated and saturated carbonyl compounds of the general formula I

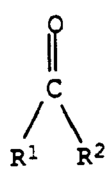

**I**

where

$R^1$ is buten-1-yl or but-1-yl, which may be substituted in the 1-, 2-, 3- and/or 4-position by up to three $R^2$ or $R^3$ radicals,

$R^2$ is hydrogen or $C_1$-$C_{20}$-alkyl, which may carry up to three of the following substituents $R^3$:
$C_3$-$C_{12}$-cycloalkyl; phenyl; naphthyl; a 5- or 6- membered aromatic or nonaromatic heterocyclic ring; $C_2$-$C_6$-alkenyl; $C_2$-$C_6$-alkynyl; $C_1$-$C_{12}$-alkoxycarbonyl; $C_1$-$C_{12}$-acyloxy; amino; mono-$C_1$-$C_4$-

alkylamino; di-$C_1$-$C_4$-alkylamino; $C_1$-$C_{12}$-acyl; di($C_1$-$C_4$-alkyl)phosphonyl; hydroxyl; cyano; $C_1$-$C_4$-alkoxy; $C_1$-$C_4$-alkylthio; and 5- or 6-membered cycloalkyl whose ring carbon atoms may be replaced by nonadjacent oxygen or sulfur atoms,

which comprises reacting a 2-formyldihydropyran derivative of the general formula II

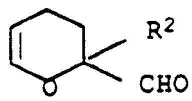

II

or a derivative of II which is substituted in the 3-, 4-, 5- and/or 6-position by up to three $R^2$ or $R^3$ radicals

a) at from 100 to 400°C in the presence of a metal of group VIII of the periodic table or in the presence of a compound of such a metal as catalyst, to give the unsaturated carbonyls, and

b) hydrogenating the unsaturated carbonyl compounds obtainable in step a) in a conventional manner to give the saturated carbonyl compounds.

## Revendications

1. Procédé de préparation de composés carbonylés, insaturés et saturés, qui répondent à la formule générale I ci-dessous

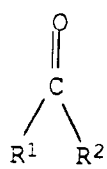

I

dans laquelle

$R^1$ représente un radical butène-l-yle ou un radical but-1-yle, qui peut porter jusqu'à trois des restes $R^2$ ou $R^3$ en tant que substituants en positions 1, 2, 3 et/ou 4 et

$R^2$ désigne les substituants qui suivent :
un atome d'hydrogène ou un radical alkyle en $C_1$-$C_{20}$, qui peut porter jusqu'à trois de substituants $R^3$ qui suivent :
cycloalkyle en $C_3$-$C_{12}$, phényle, naphtyle, hétérocycles aromatiques ou non aromatiques, pentagonaux ou hexagonaux, alcényle en $C_2$-$C_6$, alcynyle en $C_2$-$C_6$, alcoxy($C_1$-$C_{12}$)carbonyle, acyloxy en $C_1$-$C_{12}$, amino, monoalkyl($C_1$-$C_4$)amino, di-alkyl ($C_1$-$C_4$)amino, acyle en $C_1$-$C_{12}$, di-(alkyl-$C_1$-$C_4$)-phosphonyle, hydroxyle, cyano, alcoxy en $C_1$-$C_4$, alkylthio en $C_1$-$C_4$, cyloalkyle pentagonal ou hexagonal, dont les atomes de carbone cycliques peuvent être remplacés par des atomes d'oxygène ou de soufre non adjacents,

caractérisé en ce qu'un dérivé du 2-formyl-dihydropyranne de la formule générale II ci-dessous

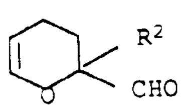

II

ou des dérivés du composé II substitués par jusqu'à 3 des restes $R^2$ ou $R^3$ en positions 3, 4, 5 et/ou 6

a) est mis à réagir, en vue de l'obtention des composés carbonylés insaturés, en présence d'un métal du huitième groupe du système périodique, ou d'un composé d'un métal de ce genre servant de catalyseurs, à des températures de 100 à 400°C et

b) on hydrogène, en vue de l'obtention des composés carbonylés saturés, les composés carbonylés insaturés obtenus conformément à l'étape a), de manière en soi connue.